# EUROPEAN PATENT APPLICATION

(11) **EP 3 388 753 A1**
(43) Date of publication of application: **17.10.2018**
(21) Application number: 15910648.3
(22) Date of filing: 17.12.2015
(51) Int. Cl.: F24F 6/10, A61L 9/03, B01D 47/02

(54) **HIGH-EFFICIENCY SYSTEM AND DEVICE IN MASS TRANSFER**

(71) Applicant: Mizrahi Aksiyote, Aldo Adolfo, 08240 Ciudad de México (MX); Higareda Juárez, Carlos, Ciudad de México (MX); Ramos De La Fuente, Rubén, Ciudad de México 04100 (MX)
(72) Inventor: RAMOS DE LA FUENTE, Rubén, Ciudad de México 04100 (MX)
(74) Representative: Padial Martinez, Ana Belen
(86) International application number: PCT/IB2015/059704
(87) International publication number: WO 2017/103657

(57) **Abstract**

The invention relates to a method, device and system, by means of which the temperature of liquid can be controlled, via solid surfaces which are humidified and supplied with an airflow, as well as acquiring moisture via being in contact with a water source, which water source, as well as said surfaces, tend to cool down, and in doing so, same and any equipment subject to the same laws and/or phenomena will lose efficiency due to the change in temperature of the liquids involved in the humidification process.

## Description

### Field of the Invention

The present invention relates to a system, method and apparatus for transferring vapor molecules from a liquid to a gas stream. Particularly, it refers to a system and apparatus for increasing humidification and filtration of air and water pollutants, and solutes dissolved in water. Specifically, a system and apparatus for transferring an aqueous mass to a specific air stream, having solid surfaces and/or aqueous membranes is described.

The present invention will be described as a system for enhancing mass transfer, wherein liquid (water) molecules are transferred by means of mass transfer to a gas stream (air), in contact with the liquid and said solid surfaces.

### Background of the Invention

Liquids have a physicochemical property called vapor pressure. Such property determines the equilibrium between the liquid-vapor phases of a liquid. By such property, a liquid will always tend to establish equilibrium between the liquid and vapor phases.

The evaporation phenomenon takes place when vapor is generated as a result of the equilibrium between the liquid-vapor phases. If a gas stream absorbs vapor in equilibrium with the liquid phase, fresh vapor will be generated to reestablish the liquid-vapor equilibrium.

Mass transfer is a phenomenon that has many applications, as in the desalination plants or environmental control systems, to name just a couple of examples.

In the case of desalination plants, the objective is to separate and remove salt from seawater to obtain drinking water. Seawater contains mineral salts dissolved therein, which is not potable for human consumption. Even, drinking large quantities of seawater can cause death. About 97.5 % of the planet's water is salty, and only an amount below 1 % is suitable for human consumption, therefore making seawater potable is one of a number of possible solutions to drinking water shortage. Since some time now, and particularly in Middle East countries desalination plants have been implemented to produce drinking water, however, the process is costly, and it is relatively seldom used. Currently there is a production of over 24 million cubic meters per day of desalinated water all over the world. The main disadvantage of the current desalination plants is that during the process of salt extraction from water, saline residues and polluting substances that endanger flora and fauna are produced. However, the main problem of such desalination process is the high-energy consumption cost, either through a reverse osmosis, distillation, freezing, flash evaporation, hydrates formation or electrodialysis process. It is known that the average cost of the most efficient desalination process per cubic meter of drinking water is about 5 to 15 dollars.

In this respect, there is a plurality of patents related to desalination processes and plants. For example, the Chinese patent application describes to an electrodialysis seawater desalination apparatus using a means of electromagnetic separation having upper and lower electrodes connected with a magnetic pole and a vertical outlet connection tube allowing water to flow through the pipe.

On the other hand, the Chinese utility model CN203922783U relates to a salt removal electromagnetic device, having an evaporation tank provided with a reservoir body provided with a vapor outlet port, and a controller connected to the reservoir body, wherein outlet vapor port ends are connected to two pipeline units. The disadvantage of said documents is that the evaporation process does not take advantage of the potential of applying mass transfer to that purpose.

In the mass transfer process, an airstream encounters the vapor in equilibrium with the liquid phase, the equilibrium is broken, and new vapor is generated to restore said equilibrium, where the air generated is humid air.

Said mass transfer process is applied to some evaporation humidifiers.

However, some humidifiers make use of other processes using a humidified material, which has the disadvantage that said material can be a source for the creation of fungus, algae and aerobic germs.

The United States Patent No. US 5,945,038 relates to an evaporative humidifier comprising an absorbent material where a portion of the humidification material is submerged, and the upper portion is exposed to air, in said patent the use of a float valve to control the water supply is also described.

Boiling humidifiers mix an air stream with a steam stream obtained from boiling water.

Said evaporators has the advantage of eliminating all kind of microorganisms, however they generate a "white powder" consisting of insoluble salts and minerals from water that are entrained by such steam streams. On the other hand, evaporation humidifiers have the disadvantage of consuming a great deal of energy to boil water. Moreover, said humidifiers do not provide means for eliminating pollutants contained in the air stream.

Warm mist humidifiers operate with steam near dew point. Steam is cooled down just before contacting the air stream, in such a way that a gas mixture of steam, small water droplets and air is obtained. Therefore, such equipment has the disadvantage of retaining air pollutants. Also, said mist humidifiers are not provided with means to remove pollutants present in the air stream.

In cold mist humidifiers, water or an aqueous liquid is sprayed and then mixed with air. Said equipment has the disadvantage of retaining pollutants and microorganisms in the humid air stream.

Such humidifiers are also not provided with means to remove pollutants contained in the air stream.

Ultrasonic humidifiers use high frequency vibrations to spray and evaporate water. Nevertheless, they have the disadvantage of requiring costly maintenance. Furthermore, such humidifiers are also not provided with means to remove pollutants contained in the air stream.

As indicated above, the boiling, the warm mist, the cold mist, and ultrasonic humidifiers do not remove the pollutants contained in air streams so that in such humidifiers, the pollutants are entrained along with the humidified air stream.

On the other hand, in the case of evaporation vaporizers, even when dust in the humidified material is removed by filtration, said dust in contact with the humidified material results in the occurrence of fungus, algae and aerobic germs. Also, over time, the dust accumulated on the humidified material has a tendency to block air circulation through the humidified material, thus affecting transfer of water to the vapor stream.

In order to remove dust, different types of filters are known. Also, the use of foam to control dust content in mine environments is known, for example in coal mines. Said foam is generated by ejectors, where it is mixed with an air stream and water stream at high velocity.

Patents No. US 4,000,992 and US 4,400,220 of January 4, 1977 and August 23, 1983, describe a system for removing dust by using bubbles in a cyclone.

Finally, Mexican Patent 264635, developed by the present applicant, refers to a system and apparatus for transferring a mass from the liquid phase to the gas phase while removing pollutants characterized by comprising a plurality of liquid membrane generating cells, where said liquid membranes are collapsed by contact with a gas stream, the collapsed liquid material covers the suspended particles and removes them via decantation, where the membrane cells also increase the speed of the gas stream and cause said stream to impinge on the surface of the liquid at an angle of 45°. However, said evaporator has some disadvantages regarding an upper threshold in mass transfer.

Nevertheless, said systems suffers from cooling on the contact surfaces, as well as in the water mirror (tank, reservoir, container of liquid) that feeds the solid surfaces in contact with the airflow that absorbs humidity through said systems; this is due to a phenomenon called "wet bulb", where, as the liquid is cooled down, its aqueous molecules tend to compact themselves by a natural temperature phenomenon (with the extreme being the formation of ice, where it becomes totally solidified); this cooling of the aqueous molecules reduces dramatically the evaporation capabilities of all the known cold evaporation systems using contact surfaces.

The present invention seeks to provide a high-efficiency system and apparatus for transferring vapor molecules from a liquid to a gas stream, for use in eliminating particles such as dust and pollutants from a humidified air stream and, additionally, removing metals, particles and salts in the liquid (water), thus substantially overcoming the problems associated with the abovementioned systems and apparatuses. The increase in efficiency is achieved by inhibiting the "wet bulb" phenomenon in the liquid mirror (water), wherein said system, method and apparatus of the present invention is based on one or several elements allowing for increasing the liquid temperature by using contact surfaces, thus increasing humidification up to 1000 %.

For carrying out the present invention the characteristics of aqueous membrane cells (bubbles) generated by using a disk were studied, where the effect of temperature on said disk and liquid was studied and verified, as well as the characteristics of the aqueous membrane cells (bubbles) generated by using a disk, considering the airflow velocity and particularly the size of the holes on the disk (membrane cells). The results showed that the efficiency in transferring liquid molecules (water) in a gas flow (air) increases according to the increase in temperature of the membrane cell on the disk, and the liquid. Wherein, the air bubble column achieves the highest efficiency in comparison to a conventional humidifier.

### Object of the Invention

According to the present invention, the main object is to provide a high-efficiency system and apparatus for transferring vapor molecules from a liquid to a gas stream.

A second object being the invention of a high-efficiency mass transfer system and apparatus allowing for controlling the temperature by means of solid surfaces that are humidified and subjected to an airflow, avoiding the wet bulb phenomenon.

A third object of the invention is a high efficiency mass transfer system and apparatus that eliminates dust and pollutants from a humidified air stream without generating fungus, algae and aerobic germs.

A fourth object of the invention is a high-efficiency mass transfer system and apparatus that eliminates solutes dissolved in water such as salts, metals or any other particle in the water.

### Brief Description of the Figures

For a detailed description of the invention, in the following, reference will be made to the accompanying drawings, in which:
Fig. 1 shows the membrane generation means of the high efficiency mass transfer system and apparatus of the present invention.
Figs. 2A and 2B show a detail of the elements comprised by each disk of the high efficiency mass transfer system and apparatus of the present invention.
Fig. 3 shows an assembly plate of membrane disks of the high efficiency mass transfer system and apparatus of the present invention.
Fig. 4 corresponds to a view of the membrane generation means of the system and apparatus for transferring vapor molecules from a liquid of the present invention.
Fig. 5 shows an exploded view of the high efficiency mass transfer system and apparatus of the present invention.
Figure 6 is a lateral sectional side view of the mass transfer system with solids capture via electromagnetic field induction of the present invention.

### Detailed Description of the Invention

As it is well known, the cold evaporation equipment using contact surfaces passes water (evaporative liquid) through solid or semi-solid surfaces, which are subjected to an airflow with the capability of absorbing humidity, that means that its relative humidity is less than 100 %, and while exchanging humidity on the humid surfaces, it absorbs part of said humidity, generating evaporation (an increase in relative humidity of the dry air that passes through the system).

In the already known systems, the contact surfaces and the liquid are subjected to an air flow, then cooled down by the "wet bulb" phenomenon, and when they are cooled down due to said phenomenon efficiency is lost, because molecules agglomerates together due to a reduction in temperature.

Particularly, the present invention relates to a method, apparatus and system, by means of which the temperature of a liquid can be controlled, via solid surfaces which are humidified and subjected to an airflow, which becomes humid by contacting a water source; said water source, as well as said surfaces, tend to warm up, and when this happens, its efficiency increases because a change in temperature of the contact surfaces, as well as in the liquid involved in the humidification process is inhibited.

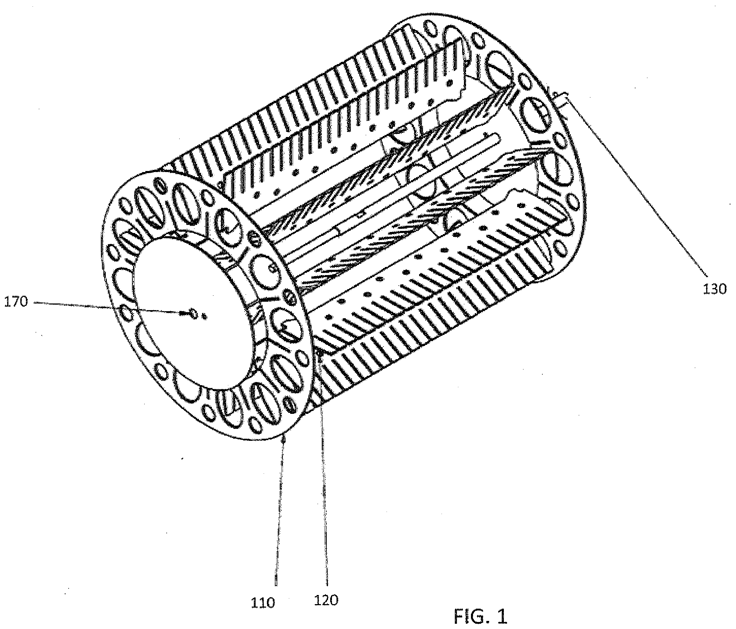

According to figure 1, the high efficiency mass transfer system of the present invention comprises a means for generating liquid membrane cells (100) comprising a plurality of disks (110) arranged on assembly plates (120) assembled on a shaft (130) for rotation, where said disks (110) are disposed parallel to an equidistant distance from each other forming a cylinder arrangement of aqueous membrane generating disks (110).

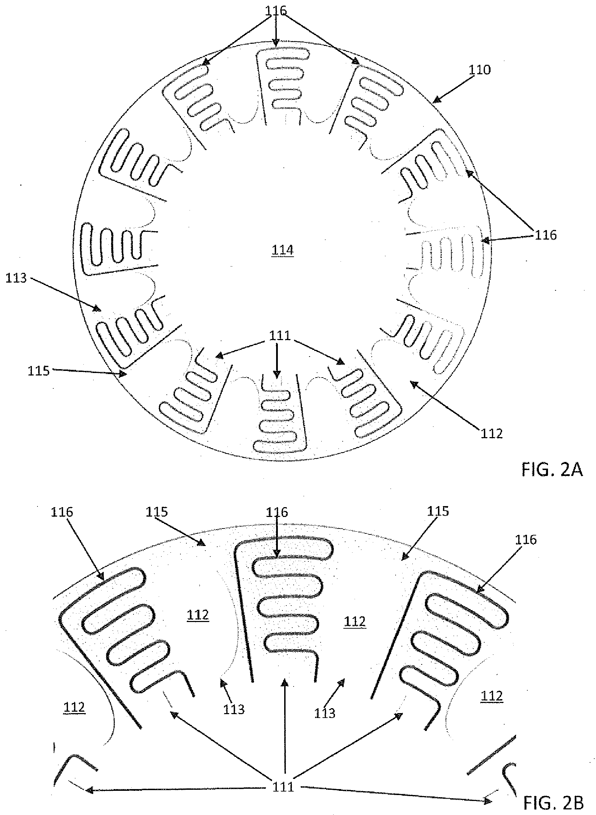

Fig. 2 illustrates a detail of a disk (110) from the plurality of disks comprising membrane generation means (100).

Each disk (110) has a hollow center (114) comprising a plurality of grooves (111) disposed on the perimeter thereof, a solid surface (115) having a plurality of holes (112) arranged with equidistant spacing from each other. Particularly, each hole (112) has a perimeter (113) by which aqueous membranes are generated, in the present description said perimeter will be referred to as membrane cells.

As illustrated in Fig. 2, said holes (112) have an oval shape and each oval perimeter is undulated, in order to provide a larger contact surface to favor liquid membrane formation, where the shape of the hole (112) and the shape of the perimeter of said holes (112) are suitable for forming a liquid membrane.

In reference to figures 1 and 2, the disks (110) of the membrane generation means (100) form liquid membranes within said holes (112) and the gaps between the disks (110), therefore there are also liquid membranes present in the gaps between disks (110) promoting the mass transfer process.

The liquid diffuses through the membranes with a gas stream (mass transfer), causing the outlet air to become modified, wherein the liquid diffusion depends on the membrane diameter within the holes (112), the velocity of the aqueous membrane (membrane-liquid ratio in a volume of air), the water temperature, the temperature of the disk (110), which leads to an increase in the mass transfer coefficient.

Turning to Fig. 2, each disk (110) comprises heat generation means (116) comprising at least one conducting element located between the holes (112) on the solid surface (115) of each disk (110), where the shape of said heat generation means (116) is wavy and undulated, however it is not limited to said shape, forming a plurality of electrical resistors connected in series or parallel on said solid surface (115) of disk (110). However, the configuration, shape and connection of the heat generation means (116), as seen in Fig. 2, is not limited to said configuration, therefore it is understood that alternative arrangements between the holes (112) and the heat generation means (116) and disks (110) having different regular and irregular patterns, which fall between the scope of the present invention, can be used.

The heat generation means (116) are electrically energized by a control element controlling the current flow through the conducting element. By using a control means and temperature sensors, the temperature of the electrical resistors forming said conducting element is controlled, whose objective is to increase the point temperature within the mass transfer zone in order to increase the efficiency of the device.

Thus, the temperature of disks (110) is controlled by using the heat generation means (116) on their solid surfaces (115) which become humid and then are subjected to an airflow, which in turn becomes humid by being in contact with a liquid source; where said disks (110) will transmit heat to the liquid source. Temperature is kept and controlled in the system in such a way that, the "wet bulb" phenomenon is inhibited in the liquid source mirror, leading to an increase in the mass transfer efficiency and performance of up to 1000 %.

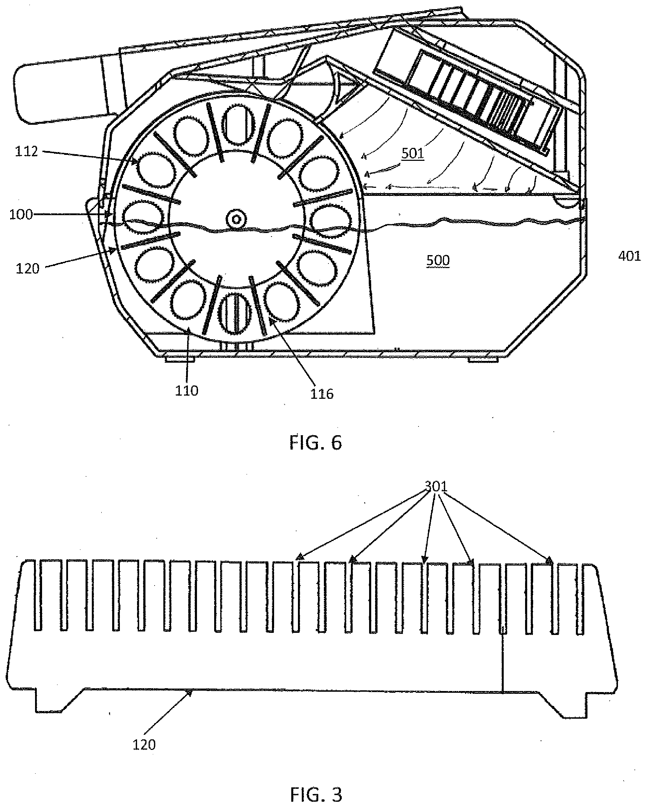

The assembly plate shown in Fig. 3 consists of a rectangular assembly plate (120) having slots (301) thereon, where said assembly plate (300) has a comb-like shape. The slots (111) of disks (110), not shown, are coupled in the slots (301) of the assembly plate (120) to form the membrane generation means, which has a structure similar to a cylinder.

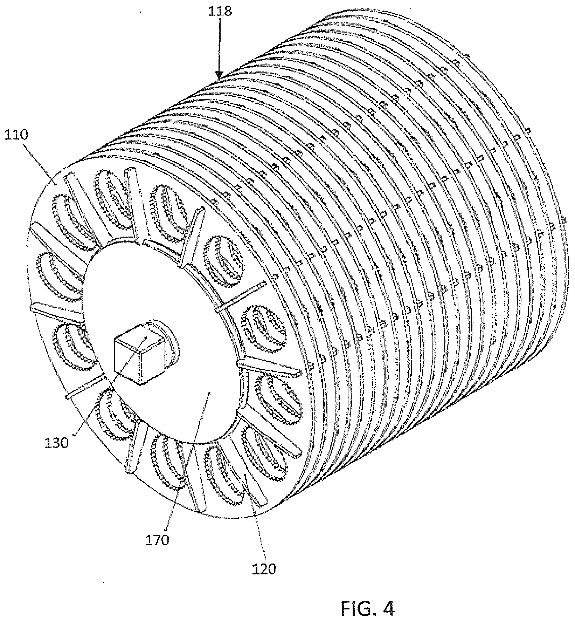

As will be evident to one skilled in the art, said disks (110) can have any shape, for example, a polygonal shape. With reference to figures 2 and 4, the hollow center (114) of the disks (110) defines a chamber (117) in the interior of the plurality of membrane cells (118) in a cylinder arrangement. A pair of caps (170) cover the hollow centers (114) of the side disks (110) of said cylinder arrangement of said membrane generation means (100), thus preventing a gas flow from entering into the center of the membrane generation means (100).

The membrane cells (118) are formed in the spaces between the solid surfaces (115) of said disks (110) and the assembly plate (120). Said cells have the shape of an irregular cube with one a widened face. The plurality of disks (110) is made of any metallic material that allows heat transmission as well as formation of an aqueous membrane.

The heat generation means (116) heat up the disk (110) to a predetermined temperature thus evaporating the liquid impregnated on the solid surface (115) and allowing rupture of the aqueous membranes between the disks (110) and the membrane cells (112) that are formed between said disks (110) by means of an injected gas flow.

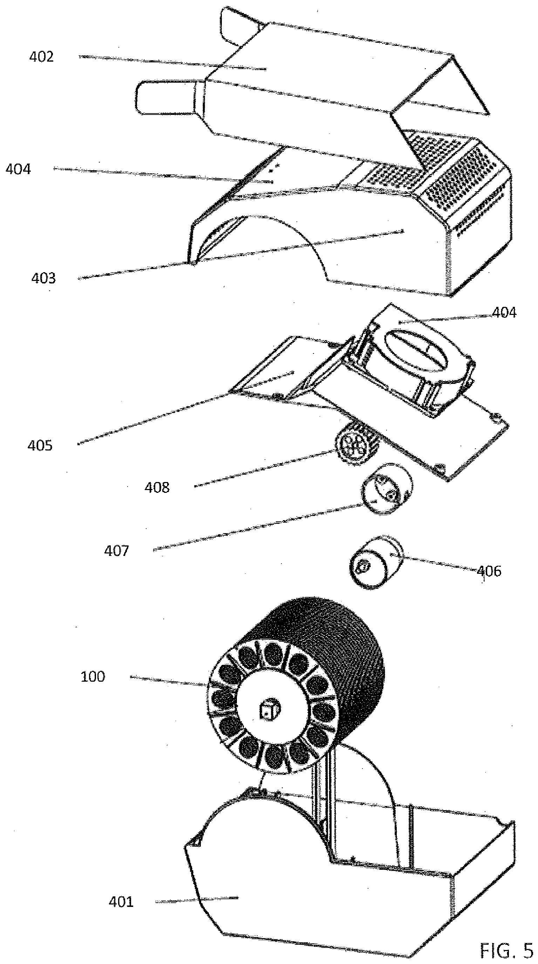

According to figure 5, the high efficiency apparatus for transferring vapor molecules from a liquid to a gas stream comprises a base (401) and a first cover (402) that constitute the housing of the apparatus, which can be made of any material, for example, metal or plastic, wherein the base (401) contains the liquid to be treated by the system. The base (401) may comprise an arrangement of at least one electrical resistor in order to make efficient the heating of the liquid to be treated; a second cover (403) has supply grooves (400) thereon for supplying gas or air, and ejection grooves (405) for ejecting gas from the apparatus.

The air convection means (404) comprise any means to force air or gas convection inside the system. According to Figs. 5 and 6 said means is as an axial fan mounted on an inner deflector (405), however, any system that generates an airflow can be used, for example, a plunger, turbine, radial fan, blower, compressor, etc. Alternatively, an external air stream can be used, for example, a stream from a pipeline. The air or gas flow can be intermittent or continuous.

A motor (406) is mounted on the base (407), generating a rotary motion and transmitting it to the membrane generation means (100) through the mechanical coupling to a toothed wheel or gear (408).

According to figure 6, the air injected by the air convection means (404) is forced to pass through the membrane generation means (100) comprising a plurality of disks (110) having each disk heat generation means (116), which are illustrated in detail in figures 1 to 3. Furthermore, the heat generation means (116) heats the liquid (500) contained in the base (401) of the high efficiency apparatus for transferring vapor molecules from a liquid to a gas stream (501).

The disks (110) rotate continuously or intermittently, so that the membrane generation means (100) is immersed in and emerged from the liquid (500), forming liquid membranes within the holes (112) of each disk (110) that constitutes the membrane generation means (100), additionally liquid membranes are formed between the space between each disk (110).

Therefore, once the air injected by the air convection means (404) passes through the disks (110), a portion of the air is deflected due to the geometry of the membrane generation means (100) and location of the comb-like assembly plates (120), causing deviation of air. The air pressures inside the membrane generation means (100) zone are homogeneous.

The vortices generated in the assembly plate (120) zone, cause the air to recirculate in those spots, this can be beneficial as it is a requirement that the flow passing through the disks (110) must be turbulent; and what the vortex values mean is a turbulent flow with spiral rotation. The injected air forms a plurality of vectors where the air flows directly towards the disks (110) and the assembly plates (300) with the aid of a deflector additionally installed (not shown in the figure). Therefore, the criterion of a greater pressure exerted on the disks (110) will be fulfilled and rupture of the membranes ensured.

The disks (110) are heated to a predetermined temperature through the heat generating means (126), which are partially immersed in the base (401) containing a liquid (500) up to a certain level of liquid. The disks (110) of the membrane generation means (100) transfer a portion of the heat generated to the liquid (500). As illustrated in Fig. 6, due to the rotation of the membrane generation means (100), the disks (110) are immersed into a position A, where the liquid of the base (401) is heated to a certain temperature by means of the heat generators (126), since the liquid wets part of the disks (110) .

Thereafter, the disk (110) rotates to position B, where half of the membrane holes (112) that were immersed now emerge, and part of the liquid (500) drains back to the container (401). However, due to the surface tension of the liquid, aqueous membranes are generated in each hole (112) emerging from the liquid surface of the base (401).

At least one aqueous membrane is formed from the liquid in the membrane generation means (100), and it is impinged with the airflow injected by the air convection means (404). The membrane collapses spraying itself into thousands of particles. Particles suspended on air are trapped by the membrane spray and decanted.

The disk arrangement (110) of said membrane generation means (100) provides to the system and apparatus of the present invention, means for channeling, space and time to allow for the humidified particles still dispersed in the gas, to precipitate and agglutinate.

The airflow (501) directly impinges on the aqueous membranes of the membrane generation means (100), which collapse when they receive the airflow, spaying themselves into thousands of small liquid particles that were part of each membrane.

The solid particles and pollutants accompanying the gas stream are decanted as a result of the saturation they were subjected to at the time of the rupture of said membranes. This effect captures suspended particles, causing a change in their weight and the precipitation thereof.

The liquid membranes described in the present disclosure are collapsed upon contact with small particles of material, such as dust, and the portions of the collapsed membrane are capable of wetting particles of the size of one micron. In this way, particles in the air are collected and agglutinated.

Simultaneously, the liquid particles of the collapsed membrane are transferred to the air stream humidifying the same. Fragrances that alternatively can be added are also transferred to the air stream by aromatizing it. The resulting airflow from the process exits completely clean, humidifying and scenting the environment.

Spraying of the liquid by rupturing the membranes, promotes the transfer of liquid into the gas stream, furthermore by controlling the temperature of the liquid (500) the "wet bulb" phenomenon in the liquid is inhibited and mass transfer efficiency is increased. As the liquid cools down, its water molecules tend to compact themselves by a natural temperature phenomenon (with the extreme being the formation of ice, where it becomes totally solidified); this cooling of the aqueous molecules reduces dramatically the evaporation capabilities.

The membrane generation means (100), in the preferred embodiment of the invention, has been illustrated as a plurality of disks (110) forming the membrane cells in a cylinder arrangement. However, it is not limited to said form or to the number of cells, as will be evident to one skilled in the art, that said arrangement can be changed. For example, they can be arranged as a block of cells through which air circulates, with the provision that the supplying liquid means must flood or wet said block of cells. A block membrane generation cells is considered as included within the scope of the present invention.

## Claims

1. An apparatus for increasing humidification and filtration of air and water pollutants, comprising: a base and a first cover forming the housing of the apparatus, air convection means forcing air or gas convection into said apparatus; a motor mounted on a base, which generates rotary motion and transmits it to a membrane generation means, characterized that the membrane generation means comprise an arrangement of at least one disk having a hollow center, a plurality of grooves arranged on its perimeter, a solid surface which has a plurality of holes disposed on the area thereof arranged equidistant from each other and heat generating means arranged between the holes and on the solid surface.

2. The apparatus for increasing humidification and filtration of air and water pollutants according to claim 1, wherein said apparatus further comprises an upper cover.

3. The apparatus for increasing humidification and filtration of air and water pollutants according to claim 1, wherein said apparatus further comprises at least one air deflector.

4. The apparatus for increasing humidification and filtration of air and water pollutants according to claim 1, wherein said heat generation means (116) is electrically energized by a control element controlling the current flow through the conducting element, controlling the temperature of the electrical resistor that forms said conducting element.

5. The apparatus for increasing humidification and filtration of air and water pollutants according to claim 1, **characterized by** further comprising a second cover having gas/air supply grooves and filtered gas/air ejection grooves (405) from of said apparatus.

6. The apparatus for increasing humidification and filtration of air and water pollutants according to claim 1, wherein said air convection means comprise means for forcing air convection inside the system.

7. The apparatus for increasing humidification and filtration of air and water pollutants according to claim 1, wherein said air convection means can be selected from an axial fan, a plunger, a turbine, a radial fan, a blower, a compressor, and an intermittent or continuous external airstream, or airstream from a pipeline.

8. The apparatus for increasing humidification and filtration of air and water pollutants according to claim 1, wherein said holes on each disk have an oval shape.

9. The apparatus for increasing humidification and filtration of air and water pollutants according to claim 8, wherein the perimeter of each hole on each disk has a toothed shape.

10. The apparatus for increasing humidification and filtration of air and water pollutants according to claim 1, wherein said assembly of membrane generation means is a cylindrical arrangement.

11. The apparatus for increasing humidification and filtration of air and water pollutants according to claim 1, wherein said assembly of heat generation means are cables of a conducting material.

12. The apparatus for increasing humidification and filtration of air and water pollutants according to claim 1, wherein the shape or arrangement of the heat generation means is undulated on said solid surface of each disk.

13. A method for increasing mass transfer in an apparatus for increasing humidification and filtration of air and water pollutants, **characterized in that** it comprises the steps of:
heating the solid surface of at least one disk comprising the membrane generation means, through at least one heat generation means disposed on the disk;
immersing at least one of said plurality of disks having oval shaped grooves forming membrane cells in the liquid;
transferring to the liquid a portion of the heat generated by the at least one heat generation means disposed on the disk;
controlling the temperature of the liquid phase in the apparatus by a control means, to inhibit the wet bulb phenomenon in the liquid, thus increasing the mass transfer efficiency;
forming a liquid membrane through at least one oval-shaped groove, when at least one oval-shaped groove passes through said liquid;
collapsing the liquid membrane by contacting the liquid membrane with a gas stream injected therein;
ejecting the gas stream, and removing particles from the water which is evaporated, and solids and pollutants from the gas that is injected.

14. A system for increasing mass transfer in an apparatus for increasing humidification and filtration of air and water pollutants, **characterized in that** it comprises: liquid membrane generation means comprising an arrangement of at least one disk having a hollow center comprising a plurality of grooves arranged on its perimeter, a solid surface having a plurality of holes disposed equidistant one from another on its surface, and heat generating means arranged between the holes and on the solid surface.
